# EUROPEAN PATENT SPECIFICATION

(11) **EP 2 170 420 B1**
(45) Date of publication and mention of the grant of the patent: **03.05.2017**
(21) Application number: 08779690.0
(22) Date of filing: 19.06.2008
(51) Int. Cl.: A61L 29/12, A61L 29/14, A61M 25/00, A61L 29/08

(54) **MULTIPLE WALL DIMENSIONALLY RECOVERABLE TUBING FOR FORMING REINFORCED MEDICAL DEVICES**
DIMENSIONAL WIEDERHERSTELLBARER MULTIWAND-SCHLAUCH ZUR FORMUNG VON VERSTÄRKTEN MEDIZINPRODUKTEN
TUBAGE POUVANT REPRENDRE SES DIMENSIONS À PAROIS MULTIPLES POUR FORMER DES DISPOSITIFS MÉDICAUX RENFORCÉS

(30) Priority: 19.06.2007 US 820266
(43) Date of publication of application: 07.04.2010
(73) Proprietor: TE Connectivity Corporation, Berwyn, PA 19312 (US); Quast, Douglas A., Lakeville, MN 55044 (US)
(72) Inventor: QUAST, Douglas, A., Lakeville, MN 55044 (US); PIESLAK, George, J., Atherton, CA 94027 (US); DEVINS, David, E., Minnetonka, MN 55345 (US); GLOVER, Leon, C., Los Altos, CA 94024 (US); IMPERIALE, Jim, J., Fremont, CA 94538 (US); ALVERNAZ, Tony, G., Bethel Island, CA 94511 (US)
(74) Representative: Johnstone, Douglas Ian
(86) International application number: PCT/US2008/007721
(87) International publication number: WO 2008/156841

(56) References cited:
- US-A- 4 817 613
- US-A1- 2005 005 989

## Description

The present invention is directed to dimensionally recoverable tubing. In particular, the present invention is directed to multiple wall, e.g. dual wall, dimensionally recoverable tubing for forming reinforced polymer tubing.

Single layer dimensionally recoverable tubing for a variety of applications may be formed by known processes. For example, U.S. Pat. No. 3,086, 242 to Cook et al, and U.S. Pat. No. 3,370,112 to Wray, disclose processes and apparatus for expanding tubing by employing a pressure differential between the inside and outside of the tubing. Cook et al. and Wray describe processes for producing dimensionally recoverable tubing by crosslinking and expanding a tube of polymeric material. In both cases, the tubing is expanded by employing a pressure differential between the inside and outside of the tubing. To form the heat-recoverable tubing, the tubing is extruded or otherwise suitably formed and crosslinked. After the tubing is crosslinked, the tubing is then heated to a temperature equal to or above its crystalline melting temperature or temperature range so as to melt the crystalline structure in the material. While the tubing is at the elevated temperature, a pressure differential is imparted across the tubing wall to expand the tubing. After subjecting the tubing to this differential pressure, the tubing is then passed through a cooling zone to cool the tubing to a temperature below the crystalline melting temperature or range to form the dimensionally recoverable tubing. The pressure differential may be imparted by continuously supplying air to the interior of the tubing, while applying ambient or sub-ambient pressure outside the tubing. Upon re-heating, the tubing will recover to the configuration it had when crosslinked.

Medical devices, such as catheters, generally require reinforcing material to provide the necessary mechanical and chemical properties, including resiliency, flexibility, lubricity, and insulation, as well as resistance to the environment within the human body, useful in catheter applications. Current manufacturing methods for catheters have included a process wherein layers of uncrosslinked polymer, such as polyether block amide polymer or polyester elastomer, are disposed within an expanded fluorinated ethylene propylene ("FEP") tube. A braid fabricated from a reinforcing material known in the art for reinforcing catheters, such as braided stainless steel fibers, is placed within the assembly. The assembly is then heated to an elevated temperature, e.g. from 182 to 218°C (360 to 425°F), which results in melting of the uncrosslinked polymer as well as contraction of the FEP tubing. As the FEP tubing contracts, the FEP tubing exerts a force on the melted uncrosslinked polymer, driving the polymer onto the braid, which consolidates the assembly. After the assembly is consolidated, the FEP tubing is removed and discarded and the resulting product is suitable for use as a catheter. This method suffers from the drawback that the use of the FEP tube, or similar device, that must be removed adds complexity and cost to the process, particularly because of yield loss due to the removal of the FEP and the potential damage to the underlying layer. Further, the high temperature required to recover the FEP requires high energy costs and specialized equipment.

US-A-2005/0005989 discloses tubing suitable for engine cooling systems, comprising an outer layer of cross-linked polyamide and an inner layer of an uncrosslinked fluoropolymer, with an intermediate adhesive layer between them. US-A-4817613 discloses the fabrication of a guiding catheter. The catheter is made from a polymer tube (PTFE) on which two torque transmitting layers are braided. The braided torque transmitting layers are made of stainless steel and Kevlar TM ribbons. A flexible casing of polyurethane is then applied as a viscous material which allows it to both coat and impregnates the braided layers. The casing is then hardened by a curing step in an oven for several hours.

There remains a need for a method and system for forming dimensionally recoverable tubing for use in reinforced medical devices that are easily fabricated, create less production scrap, and do not suffer from the deficiencies noted above.

According to a first aspect of the present invention there is provided a multi-layered dimensionally heat-recoverable tubing system comprising:
a first crosslinked polymeric layer which is an outer layer of the tubing system;
a second uncrosslinked polymeric layer disposed adjacent to the first layer; and
a reinforcing structure positioned adjacent the second layer;
characterised in that the second layer is coextruded with the first layer, in that the uncrosslinked material of the second polymeric layer has sufficient flowability to allow infiltration by the reinforcing structure and in that the first and second layers are dimensionally heat recoverable and are configured such that the reinforcing structure infiltrates uncrosslinked material of the second layer when the tubing system is dimensionally recovered.

Another aspect of the present invention includes a method for making a reinforced tubular medical device. The method includes providing a tubular first polymeric layer preferably having a crosslinking agent. An uncrosslinked tubular second polymeric layer is coextruded adjacent to and within the first layer to form a multiple layer assembly. The first layer is exposed to conditions sufficient to result in crosslinking of the first layer. The multiple layer assembly is expanded to render the multiple layer assembly dimensionally heat-recoverable. A reinforcing structure is provided adjacent to and within the second layer. The multiple layer assembly is heated to a temperature sufficient to at least partially dimensionally recover the first layer and to incorporate the reinforcing structure into the second layer. The multiple layer assembly is consolidated to form a reinforced multiple layer device.

Another aspect of the present invention provides a reinforced medical device comprising a multiple layer heat-recoverable tubing system as defined above, wherein the reinforcing structure is incorporated into the second layer.

An advantage of an embodiment of the present invention is that the dimensionally recoverable first layer provides formation and incorporation of the reinforcing structure with easier processing and reduced waste. The first layer does not require removal after final consolidation of the reinforced device, thus minimizing the amount of production scrap.

Another advantage of the present invention is that the process of forming the reinforced device may be performed utilizing readily available equipment with few process steps.

The present invention allows a higher shrink ratio than currently available with FEP. Also, this concept can be used to connect a larger shaft to a smaller shaft with one piece.

Other features and advantages of the present invention will be apparent from the following more detailed description of the preferred embodiment, taken in conjunction with the accompanying drawings which illustrate, by way of example, the principles of the invention and wherein:
FIG. 1 shows a perspective view of a dimensionally recoverable multiple layer assembly for forming a reinforced device, e.g. a medical device, according to an embodiment of the present invention.
FIG. 2 shows a perspective view, before recovery, of a dimensionally recoverable multiple layer assembly for forming a reinforced device, e.g. a medical device, with a reinforcing structure positioned therein according to an embodiment of the present invention.
FIG. 3 shows a cross-sectional view of the assembly of FIG. 2.
FIG. 4 shows a perspective view of a reinforced medical device according to an embodiment of the present invention.
FIG. 5 shows a cross-sectional view of the assembly of FIG. 4.
FIG. 6 shows a perspective view of a dimensionally recoverable multiple layer assembly for forming a reinforced medical device according to another embodiment of the present invention.
FIG. 7 shows a perspective view of a dimensionally recoverable multiple layer assembly for forming a reinforced medical device according to still another embodiment of the present invention.

The same reference numbers will be used throughout the drawings to refer to the same or like parts.

The present invention utilizes an assembly having at least one layer of a dimensionally recoverable material, preferably in the form of a tube or sheath, to provide a force, when heated, onto a uncrosslinked material to urge the uncrosslinked material over, around and/or into a reinforcing structure, to form a reinforced structure useful as a medical device. As utilized herein "crosslinked" materials and grammatical variations thereof are defined as materials that are partially or fully crosslinked or material having high degrees of chemical (e.g., polymeric) crosslinking. As utilized herein "uncrosslinked" materials and grammatical variations thereof are defined as materials that are non-crosslinked or material having low degrees of chemical crosslinking, wherein the amount of crosslinking is sufficiently low to allow flowability. For example, uncrosslinked materials preferably have sufficient flowability to permit infiltration of a reinforcing structure into the uncrosslinked material. Further "non-crosslinkable" materials and grammatical variations thereof are defined as materials having a partial or total resistance to crosslinking when exposed to conditions sufficient to cause crosslinking in crosslinkable materials.

An embodiment of the present invention includes a multiple layer system for forming reinforced medical devices, where the multiple layer system includes at least one layer of a dimensionally recoverable material. A polymeric heat-recoverable material is a dimensionally heat unstable material frequently said to possess "elastic memory". One form of heat-recoverable material includes tubular sheaths suitable for wrapping or encompassing elongated components. As is well-known to those skilled in the art, materials having the property of elastic memory are dimensionally heat unstable and may be caused to change shape and/or dimension by the application of heat. Elastic memory may be imparted to polymeric materials by first extruding or otherwise molding the polymer into a desired shape. The polymer is then crosslinked or given the properties of a crosslinked material by exposure to high energy radiation, e.g., a high energy electron beam, Co⁶⁰ gamma irradiation, or exposure to ultra-violet irradiation, or by chemical means, e.g., incorporation of a peroxide. The crosslinked polymeric material is then heated and deformed and then locked in that deformed condition by quenching or other suitable cooling. Alternatively, for some systems, the expansion may be accomplished at room temperature by using greater force to deform the polymer. The deformed material will retain its shape almost indefinitely until exposed to an elevated temperature sufficient to cause recovery. The property of elastic memory can also be imparted without actual crosslinking to materials, such as some perfluoropolymers (e.g. polytetrafluoroethylene and FEP) and polyolefins or vinyl polymers that have a sufficiently high molecular weight to give the polymer appreciable strength at temperatures below the crystalline melting point. These materials can be expanded at temperatures between the glass transition temperature and the melting point. The expansion can be from about 120 to about 600 percent, which is often much greater than can be accomplished by simply expanding in the molten (amorphous) state.

Where a simple tubular shape is desired it may be fabricated from a flat sheet of material simply by rolling it into a tube and suitably sealing the seam. Tubing may be supplied as a sheet that is rolled into position before application of heat. Recoverable articles are frequently used to cover objects having a tubular or otherwise regular elongate configuration, to provide, for example, environmental sealing protection. Where no free end of the elongate object is available, it is common practice to use a so-called wrap-around material, that is a material, typically in the form of a sheet, that is installed by wrapping it around the object to be covered so that opposed longitudinal edges overlap. In order to hold the wrap-around material around the object, a closure means may be applied to secure together the opposed longitudinal edges; although one skilled in the art will readily appreciate that, depending on the particular application, the adhesive component may be sufficient to seal the material to the object. In an example embodiment, the tubing system is a multi-layered, heat-shrinkable tube having a substantially cylindrical shape.

For example, the tubing system may be cylindrical and have a ratio of the inner diameter of the expanded tubing to the inner diameter of the recovered tubing (recovery ratio) of from about 1.2 to about 6, or much larger. The inner wall and the expanded polymeric outer jacket taken together may have a thickness of from about 0.04 mm (0.0016 inches) to about 1.25 mm (0.05 in). The inner layer may be thicker than the outer layer. Alternatively, the outer layer may be thicker than the inner layer if greater force on recovery is desired.

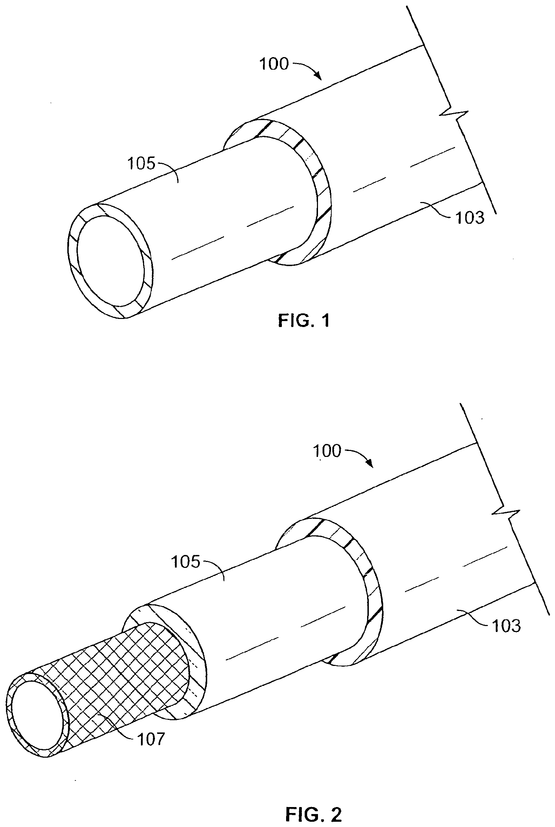

FIG. 1 shows a perspective view of a dimensionally recoverable multiple layer assembly 100 in the system for forming a reinforced medical device with portions of layers removed. In the embodiment shown in FIG. 1 the assembly 100 includes cylindrically arranged layers forming tubes of material in which the outer layer 103 concentrically encompasses the inner layer 105. The dimensions represented in FIGs. 1-7 are merely schematic and are not drawn to scale. The thicknesses of the layers present depend upon the desired properties of the reinforced medical device and vary dependent upon material compositions utilized in the outer layer 103 and the inner layer 105.

As shown in FIG. 1, the dimensionally recoverable assembly 100 includes an outer layer 103, and an inner layer 105. The outer layer 103 and inner layer 105 have been co-extruded and disposed in an adjacent relationship. "Adjacent", as utilized herein, includes positioning of layers in close proximity to and/or including layers that may have layers or materials intermediate thereto. The outer layer 103 and the inner layer 105 are preferably formed from a polymer material capable of being formed into a dimensionally recoverable structure. The outer layer 103 and the inner layer 105 may be formed of the same material or different materials. Materials for use as polymeric material are not particularly limited and may include any materials that are capable of being formed into a dimensionally recoverable structure. For example, polymers suited for use in this invention may include, e.g., polyolefins, saturated polyesters, polyamides, and polyvinyl halides, etc. In addition, elastomers such as thermoplastic elastomers, polysiloxanes, and plasticized polyvinylchloride, etc. may be used. Further, polyolefins, e.g., polyethylene, polypropylene, various copolymers of ethylene, propylene and butene; ethylene-ethylacrylate, ethylene-vinylacetate, ethylene-methyl acrylate, or ethylene-butyl acrylate copolymers in which repeat units derived from the ethylene comonomer predominate (e.g., 80-90%), and blends of such copolymers containing major portions of polyethylene itself, may be used. Alternatively, fluoropolymers such as polyvinylidene fluoride and ethylene-tetrafluoroethylene copolymer may be used. Preferred polymers for use with this embodiment include polyether block amide polymers, polyester thermoplastic elastomers and polyurethane thermoplastic elastomers. In addition, blends of polymers, such as the polymers listed above may be utilized in the formation of the outer layer 103 and/or the inner layer 105. The outer layer and/or the inner layer may be constructed of two or more segments of polymers having different physical or thermal properties, e.g. polymers of the same chemical composition but different durometers or colors. Such segments can be fused or bonded together into a unitary structure.

The outer layer 103 further includes a crosslinking agent to promote crosslinking when irradiated with an electron beam or other suitable source of energy capable of crosslinking the material of the outer layer. Suitable crosslinking agents may include any crosslinking promoter that facilitates crosslinking within the outer layer 103 when irradiated. Suitable crosslinking promoters include, but are not limited to, triallyl cyanurate, triallyl isocyanurate, N,N'-m-phenylene-dimaleimide, and multifunctional acrylates or methacrylates. In addition or alternatively, chemical crosslinking agents, such as peroxides, may be used in place of radiation crosslinking.

The inner layer 105 includes a substantially uncrosslinked elastomeric polymer. The inner layer 105 may include the polymer of the outer layer 103 or may be fabricated from a different material. The inner layer 105 is formulated with sufficient polymer and, if desired, a crosslinking inhibitor (e.g. some antioxidants), to provide resistance to crosslinking during irradiation. Crosslinking inhibitors include any materials that impart the polymer of the inner layer 105 with a resistance to crosslinking when exposed to ionizing radiation. Suitable crosslinking inhibitors include, but are not limited to, phenolic antioxidant or thioester, or aromatic disulfide. While it is preferred to substantially avoid crosslinking of the inner layer 105 for embodiments in which the inner layer must flow in contact with the reinforcing structure (not illustrated in FIG. 1), some crosslinking may be provided to the inner layer 105 during irradiation, provided the material maintains sufficient flowability to permit infiltration of the reinforcing structure.

In an alternate embodiment, the inner layer 105 may include a flow agent, e.g., a wax. The wax preferably provides additional resistance to crosslinking, particularly when in combination with the antioxidant. Suitable wax compositions for use with the present invention may include, but are not limited to low molecular weight polyethylene or polypropylene polymers or other wax polymer compositions suitable for use in a tubing layer.

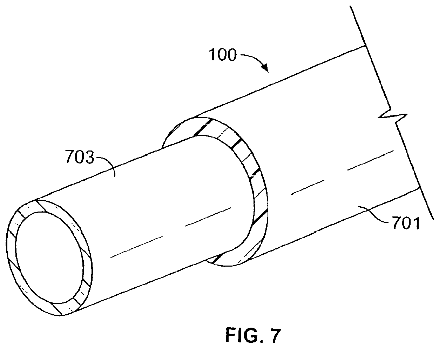

The inner layer may contain a crosslinking inhibitor to enhance the ability of the inner layer to flow. In an alternate embodiment, the inner layer 105 may contain an adhesive. If desired for a "reverse" embodiment such as that shown in FIG. 7, the adhesive for use in the inner layer 105 in this embodiment of the invention may include a crosslinkable adhesive. A preferred adhesive polymer for use in the inner layer 105 may include polyethylene copolymers (e.g., ethylene copolymers), polyamides, and combinations thereof. Specific copolymers of ethylene and one or more monoolefinically unsaturated polar comonomers have comonomers including monoolefinically unsaturated organic esters and acids copolymerizable with ethylene. Among suitable unsaturated esters are the vinyl esters of alkanoic and aromatic acids including, but not limited to, vinyl acetate, vinyl proprionate, vinyl butyrate, vinyl isobutyrate, vinyl benzoate and the like. Alkyl and aryl esters of monoolefinically unsaturated acids, such as, but are not limited to, methyl acrylate, ethyl acrylate, methyl methacrylate, ethyl methacrylate, n-butyl acrylate, phenyl acrylate, methyl crotonate, ethyl crotonate and the like may be utilized. Suitable unsaturated acids may include acrylic and methacrylic acid. Applicable copolymers include those of ethylene with one or more of the aforementioned monomers. Preferred copolymers include those of ethylene and vinyl acetate, terpolymers of ethylene with vinyl acetate and either acrylic or methacrylic acid. Another preferred copolymer includes a polymer of ethylene and ethyl acrylate. The adhesive, like the polymer composition of the inner layer 105, may be slightly crosslinked during irradiation, e.g., less than 20% crosslinking.

The inner layer 105 may include one or more of the above ingredients alone or in combination, wherein the composition may depend upon the desired properties of the resultant medical device. In addition, other additives such as color concentrates, dyes or pigments, stabilizers, fillers, crosslinking inhibitors, antioxidants, reaction promoters, lubricating agents, radiopaque fillers, or other additives may be added to the outer layer 103 and/or inner layer 105 to provide desired properties.

As discussed in greater detail above, the outer layer 103 and the inner layer 105 are formed as a dimensionally recoverable structure by known methods for rendering such polymeric materials heat-recoverable. After the outer layer 103 and inner layer 105 are formed into the multiple layer assembly 100, such as by co-extrusion into tubular geometries, the assembly is irradiated with ionizing radiation or ultraviolet radiation. Ionizing radiation may be provided by accelerated electrons, X-rays, gamma rays, alpha particles, beta particles, neutrons or other high energy radiation sources. The radiation exposure is preferably sufficient to cause crosslinking of at least the polymeric material of the outer layer 103. Radiation dosage of from 2 to 60 megarads (Mrads) may be employed to provide sufficient crosslinking to the outer layer 103. While irradiating is a preferred manner to induce crosslinking, chemical crosslinking agents may alternatively be present in the outer layer 103, wherein the crosslinking mechanism is chemical crosslinking of the polymeric material.

As discussed above, the co-extruded or otherwise formed layers are preferably crosslinked to different extents. Specifically, the outer layer 103 is preferably crosslinked to a greater extent than the inner layer 105, which is preferably non-crosslinked. As discussed above, crosslinking may be achieved by irradiating the material with a beam of high energy electrons, and the different amount of crosslinking between the outer layer 103 and the inner layer 105 is preferably provided by adding selective amounts of crosslinking promoters to the outer layer 103, antioxidants and/or crosslinking inhibitors to the inner layer 105. In another embodiment of the invention, the radiation source may be used in a manner to irradiate the outer layer 103 to a greater extent than the inner layer 105 in order to induce a greater amount of crosslinking in the outer layer 103.

In a preferred embodiment of the present invention the same polymeric material is incorporated into the outer layer 103 and the inner layer 105. The outer layer 103 is fully crosslinked after irradiation and the inner layer 105 is non-crosslinked. Further, while the system has been shown and described with respect to a dual layer system (i.e. an outer layer 103 and an inner layer 105) any number of layers may be provided. Furthermore, additional reinforcing structures 107 (FIG. 2) may be provided in any suitable location within the system, including adjacent to the outer layer 103 or the inner layer 105. The additional layers or structure may be provided in any suitable manner, including adjacent to or in close proximity to the outer layer 103 and/or the inner layer 105.

FiGs. 2-3 show the positioning of reinforcing structure 107 adjacent to the inner layer 105 in preparation of the formation of a reinforced device, such as a reinforced medical device. The material forming the reinforcing structure 107 is not particularly limited and may include any material that provides the properties, particularly when incorporated in the inner layer 105, desirable for use as a medical device. Desirable properties may include high tensile strength, resistance to kinking, high burst pressure, puncture resistance, chemical resistance, human tissue compatibility, thermal stability, non-toxicity, resistance to moisture, and the ability to sterilize via gas or radiation. For example, the reinforcing structure may include monofilament metallic or alloy material formed or woven into a braid or fabric. For example, the reinforcing structure 107 may include woven or braided stainless steel wire, PET fiber, nylon fiber, and/or aramid fiber. In addition, the reinforcing structure may take the form of elongated tapes or fiber bundles. Alternatively, the reinforcing structure 107 may comprise a non-woven sheet that has been perforated or a layer that has microscopic protrusions that allow polymer of the inner layer to interlock with the protrusions. In another embodiment, the reinforcing structure 107 is formed from woven high temperature resistant polymeric materials. In yet another embodiment, the reinforcing structure 107 may comprise a metal or polymeric fiber that is spiral-wrapped, coiled, or otherwise positioned in contact with the inner layer. The reinforcing structure such as a braid or fabric provides stiffness and resiliency to the medical device useful in the environments within the human body. The placement of the reinforcing structure 107 is not limited to the location shown in FIGs. 2-3 and may include any positioning of the reinforcing structure 107 that provides incorporation of the reinforcing structure 107 into the inner layer 105 in response to heating and contracting of the outer layer 103. The reinforcing structure may extend the entire length of the first and/or second layers, or only part of the length of the first and/or second layers, depending on the application. Two or more different types of reinforcing structures may be used in the same tubing system in order to provide specific properties.

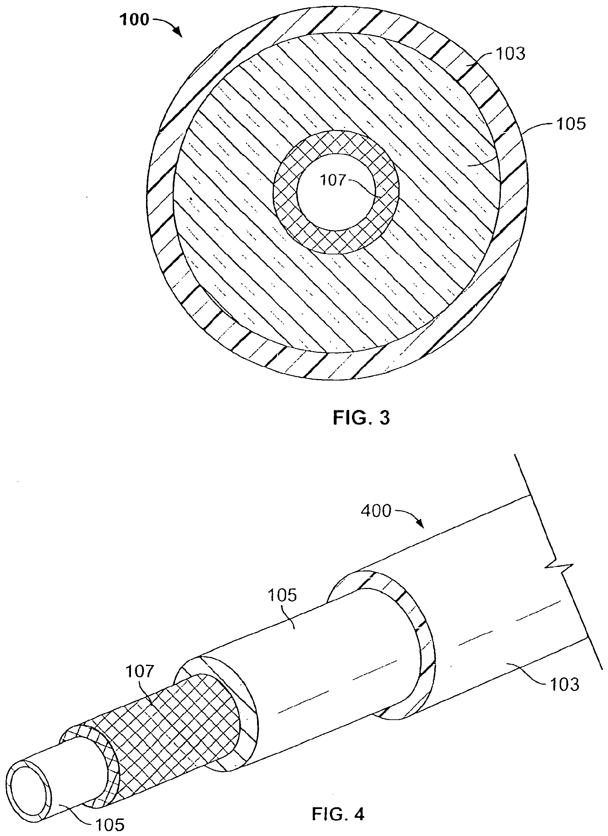

In order to form the medical device, the assembly 100 including the adjacent reinforcing structure 107, shown in FIGs. 2-3, is subject to heating to temperatures sufficient to recover the structure of at least the outer layer 103. For example, the assembly 100 may be heated in an oven or exposed to the heat from a heat gun or similar device. Suitable temperatures for heating depend on the polymer used, and may be, e.g., from 90°C (194°F) for ethylene copolymers to 250°C (482°F) for FEP or greater. The assembly is heated sufficiently to cause contracting of the outer layer 103, thereby providing a force on the inner layer 105, which is melted or otherwise flowable over the reinforcing structure 107. Upon completion of the incorporation of the reinforcing structure 107 into the inner layer 105 (see, e.g., FIG. 4), the materials are cooled and consolidated to form a reinforced device 400. In an embodiment of the invention, an inner layer 105 comprising a curable material, such as adhesive, having the reinforcing structure 107 incorporated therein is permitted to cure.

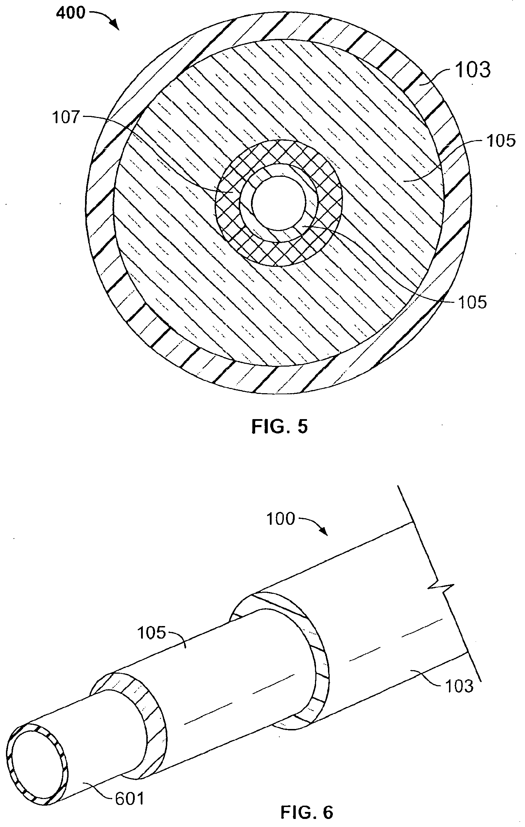

As shown in FIG. 4, the dimensionally recovered reinforced device 400 includes the outer layer 103 and inner layer 105. The outer layer 103 and inner layer 105 are preferably fully dimensionally recovered. The inner layer 105 includes the reinforcing structure 107 incorporated therein. As shown in FIG. 5, while not so limited, the reinforcing structure 107 is preferably fully encompassed by the inner layer 105 to prevent exposure of the reinforcing structure 107 to the environment inside the reinforced device 400 or outside the reinforced device 400.

The invention is not limited to the arrangement shown and described above in FIGs. 1-5 and may include additional layers. For example, as shown in FIG. 6, the present invention includes an embodiment including a dimensionally recoverable three-layer system having a crosslinked outer layer 103 and an uncrosslinked inner layer 105. The dimensionally recoverable assembly 100 further includes a crosslinked inner capping layer 601 adjacent to the inner layer 105. The outer layer 103 and the inner layer 105 include compositions, such as the compositions described with respect to outer layer 103 and the inner layer 105 above. In addition, the inner capping layer 601 includes a crosslinked composition, as described with respect to the outer layer 105 above. The inner capping layer 601 and the outer layer 103 may include compositions that are the same or may be different. In this embodiment of the invention, the outer layer 105 and the inner capping layer 601 preferably include crosslinking agents to increase the amount and rate of crosslinking.

In another embodiment, as shown in FIG. 7, the present invention includes an embodiment including another system having an uncrosslinked outer layer 701 and a crosslinked inner layer 703. The inner layer 703 includes crosslinked compositions, such as the compositions described above with respect outer layer 103. The outer layer 701 includes uncrosslinked compositions, such as the compositions described above with respect inner layer 105. The arrangement of FIG. 7 permits incorporation of a reinforcing structure (not shown in FIG. 7) into the outer layer 701 of the assembly 100, which may be accomplished using any suitable technique, including positioning the reinforcing structure adjacent to the outer layer 701 and drawing the reinforcing layer into the outer layer 701. Alternatively, the reinforcing structure may be positioned adjacent to the outer layer 701 and an additional dimensionally recoverable crosslinked layer (not shown in FIG. 7) may be provided adjacent to the reinforcing structure, wherein the dimensionally recoverable layer, when recovered urges the reinforcing structure into the uncrosslinked outer layer 701. In addition, the embodiment of FIG. 7 may include an additional inner capping layer that is not crosslinked (not shown), similar to the inner capping layer 601 shown in FIG. 6.

While the above has been shown and described with respect to tubular structures and concentric arrangements, planar or other arrangements of dimensionally recoverable materials may be utilized, wherein the material may be joined together utilizing known bonding techniques to form reinforced devices having mechanical properties desirable for use as medical devices. In addition, the reinforced structure 400, although described as being suitable for a catheter, is also configurable into catheter components, such as balloons, or other medical devices. Further the reinforced device 400 is not limited to medical applications and may include any applications that require reinforced flexible tubing. For example, the device 400 according to embodiments of the present invention includes other medical applications, such as introducers, dilators, leaders, and physiology devices (such as ablation catheters).

One embodiment of the invention includes a dimensionally recoverable multiple layer tubing assembly for forming a reinforced medical device fabricated by coextruding an outer layer and an inner layer. When the outer layer is the crosslinked layer, it may contain 0.5-5 wt% crosslinking promoter, 2-5 wt% color concentrate, 0.5-1 wt% antioxidant and the balance polymer, all weight percentages being by weight of the total composition. When the inner layer is not crosslinked, it may contain 0.5-1 wt% antioxidant, 2-5 wt% color concentrate and, optionally, 1-5 wt% crosslinking inhibitor, wherein the balance is polymer, all weight percentages being by weight of the total composition. The co-extruded assembly of the outer layer and inner layer is then irradiated with an electron beam. The assembly is then expanded via conventional expansion techniques to render the assembly dimensionally recoverable. In order to form a reinforced medical device, a reinforcing structure, e.g. a braid, is disposed within the dimensionally recoverable assembly, adjacent to the inner layer. The assembly, including the braid, is heated in an oven to a temperature greater than 120 °C (250 °F). The inner layer melts and the outer layer contracts thereby causing the inner layer to flow and incorporate the braid therein. The assembly is then permitted to cool. The resultant device includes an outer layer and a reinforced inner layer.

Another embodiment of the invention includes a dimensionally recoverable multiple layer tubing assembly for forming a reinforced medical device fabricated by coextruding an outer layer and an inner layer. The outer layer contains 0.5-5 wt% crosslinking promoter, 2-5 wt% color concentrate, 0.5-1 wt% antioxidant and the balance polymer, all weight percentages being by weight of the total composition. The inner layer contains 1-5 wt% of the total composition crosslinking inhibitor, wherein the balance is a polymeric material that has adhesive properties. The co-extruded assembly of the outer layer and inner layer is then irradiated with an electron beam. The assembly is then expanded via conventional expansion techniques to render the assembly dimensionally recoverable. In order to form a reinforced medical device, a reinforcing structure, e.g. stainless steel braid, is disposed within the assembly, adjacent to the inner layer. The assembly, including the braid, is heated in an oven to a temperature greater than 120 °C (250 °F). The inner layer melts and the outer layer contracts thereby causing the inner layer to flow and incorporate the braid therein. The assembly is then permitted to cool. The resultant device includes an outer layer and a reinforced inner layer.

The principles of the invention are further illustrated by the following examples, which should not be construed as limiting.

### EXAMPLES

Outer layer compositions were made on a 76.2 mm (3") diameter, two-roll mill that was heated to 180 °C. The outer layer (crosslinkable) compositions were made by mixing PEBAX™ polyether block amide polymer resins (available from Arkema Corporation) with 2.5% or 5.0% crosslinking promoter (triallyl isocyanurate). Plaques, 152 mm x 152 mm x 0.635 mm (6 in x 6 in x 0.025 in), were pressed from these blends in an electric press at 180°C (365 °F). These plaque samples were irradiated to 10 or 20 Mrads using a 1.0 MeV electron beam and were tested for crosslink density by conducting a test for E30 at 200°C. The E30 test measured the force at 30% elongation at 200°C, using an Instron™ tester equipped with a hot box. A sample having dimensions of 6.35 mm x 102 mm x 0.635 mm (0.25 in x 4 in x 0.025 in) was placed in the Instron tester with a jaw separation of 48.3 mm (1.9 in) and pulled at a rate of 50 mm/min. This test was conducted at 200 °C, i.e. at least 25°C above the melting point of the highest melting PEBAX™ resin. (PEBAX™ 7233 had the highest melting point of 174°C.) Tubing which expands well typically has an E30 value greater than 0.34 MPa (50 psi) and preferably an E30 greater than 0.62 MPa (90 psi). The E30 data are summarized in Table 1. The melting point of each PEBAX™ resin as measured according to ASTM D-3418, and the Shore hardness (durometer), Shore D value after 15 seconds as measured by ASTM D2240, are shown in Table 1.

**Table 1**

| Composition | | | | | | | | | | | | | | |
|---|---|---|---|---|---|---|---|---|---|---|---|---|---|---|
| Component* (weight %) | | | 1 | 2 | 3 | 4 | 5 | 6 | 7 | 8 | 9 | 10 | 11 | 12 |
| | Tₘ °C | Shore D | | | | | | | | | | | | |
| PEBAX™ 7233 | 174 | 61 | 97.5 | 95.0 | | | | | | | | | | |
| PEBAX™ 6333 | 169 | 58 | | | 97.5 | 95.0 | | | | | | | | |
| PEBAX™ 5533 | 159 | 50 | | | | | 97.5 | 95.0 | | | | | | |
| PEBAX™ 4033 | 160 | 35 | | | | | | | 97.5 | 95.0 | | | | |
| PEBAX™ 3533 | 143.5 | 25 | | | | | | | | | 97.5 | 95.0 | | |
| PFBAX™ 2533 | 133.5 | 22 | | | | | | | | | | | 97.5 | 95.0 |
| TAIC | | | 2.5 | 5.0 | 2.5 | 5.0 | 2.5 | 5.0 | 2.5 | 5.0 | 2.5 | 5.0 | 2.5 | 5.0 |

| E30 (MPa / psi) | | | | | | | | | | | | | | |
|---|---|---|---|---|---|---|---|---|---|---|---|---|---|---|
| E30 at 10 Mrads | | | 0.37 (54) | 0.75 (108) | 0.24 (35) | 0.56 (81) | 0.26 (38) | 0.51 (74) | 0.24 (35) | 0.57 (83) | 0.47 (68) | 0.61 (88) | 0.43 (63) | 0.52 (76) |
| E30 at 20 Mrads | | | 0.40 (58) | 0.85 (124) | 0.46 (67) | 0.77 (112) | 0.50 (73) | 0.78 (113) | 0.27 (39) | 0.65 (94) | 1.03 (149) | 1.44 (209) | 0.91 (132) | 1.33 (193) |

| | | | | | | | | | | | | | | |
|---|---|---|---|---|---|---|---|---|---|---|---|---|---|---|
| * PEBAX™ 7233, 6333, 5533, 4033, 3533, 2533 are polyether block amide polymers, available from Arkema Corporation; TAIC is triallylisocyanurate. | | | | | | | | | | | | | | |

The inner layer (uncrosslinked) compositions were made by mixing PEBAX™ resins with 1.0-3.0% LOWINOX™ TBM6 antioxidant (a phenolic antioxidant available from Chemtura Corporation) or with a combination of 1.0-4.0% LOWINOX ™ TBM6 antioxidant and 18.0% of EPOLENE™ C 13 wax (available from Eastman Chemical Company). Plaques, 152 mm x 152 mm x 0.635 mm (6 in x 6 in x 0.025 in), were pressed from these blends in an electric press at 180°C (365 °F). These plaque samples were irradiated to 10 or 20 Mrads in a 1.0 MeV electron beam and were evaluated in the Melt Flow Rate (MFR) test according to ASTM D 1238-04c test procedure, which is hereby incorporated by reference, Test Method for Flow Rates of Thermoplastics by Extrusion Plastometer, Procedure A, Condition 230/2.16 (230°C with 2.16 kg load). Several compositions, which had MFR values equivalent or higher than the MFR of unirradiated PEBAX™ resins, were chosen for conversion into tubing prototypes. The MFR data are summarized in Table 2.

**Table 2**

| Inner Layer Compositions | | | | | | | | | | |
|---|---|---|---|---|---|---|---|---|---|---|
| Component * (weight %) | 1 | 2 | 3 | 4 | 5 | 6 | 7 | 8 | 9 | 10 |
| PEBAX™ 7233 | 100.0 | 99.0 | 98.0 | 81.0 | 80.0 | | | | | |
| PEBAX™ 6333 | | | | | | 100.0 | 99.0 | 98.0 | 81.0 | 80.0 |
| PEBAX™ 5533 | | | | | | | | | | |
| PEBAX™ 4033 | | | | | | | | | | |
| PEBAX™ 3533 | | | | | | | | | | |
| PEBAX™ 2533 | | | | | | | | | | |
| EPOLENE™ C13 | | | | 18.0 | 18.0 | | | | 18.0 | 18.0 |
| LOWINOX™ TBM6 | | 1.0 | 2.0 | 1.0 | 2.0 | | 1.0 | 2.0 | 1.0 | 2.0 |
| MFR (g/10 min): 0 Mrads | 24 | 13 | 14 | 22 | 22 | 26 | 21 | 23 | 30 | 32 |
| MFR: 10 Mrads | 15 | 41 | 50 | 42 | 50 | 7 | 43 | 56 | 42 | 52 |
| MFR: 20 Mrads | 2 | 65 | 85 | 43 | 70 | 1 | 48 | 74 | 36 | 55 |

| Component * (weight %) | 11 | 12 | 13 | 14 | 15 | 16 | 17 | 18 | 19 | 20 |
|---|---|---|---|---|---|---|---|---|---|---|
| PEBAX™ 7233 | | | | | | | | | | |
| PEBAX™ 6333 | | | | | | | | | | |
| PEBAX™ 5533 | 100.0 | 99.0 | 98.0 | 81.0 | 80.0 | | | | | |
| PEBAX™ 4033 | | | | | | 100.0 | 99.0 | 98.0 | 81.0 | 80.0 |
| PEBAX™ 3533 | | | | | | | | | | |
| PEBAX™ 2533 | | | | | | | | | | |
| EPOLENE™ C13 | | | | 18.0 | 18.0 | | | | | |
| LOWINOX™ TBM6 | | 1.0 | 2.0 | 1.0 | 2.0 | | 1.0 | 2.0 | 1.0 | 2.0 |
| MFR (g/10 min): 0 Mrads | 27 | 24 | 27 | 34 | 37 | 22 | 22 | 24 | 35 | 37 |
| MFR: 10 Mrads | 6 | 43 | 62 | 28 | 51 | 18 | 30 | 43 | 25 | 33 |
| MFR: 20 Mrads | 0.4 | 49 | 97 | 28 | 46 | 2 | 13 | 45 | 10 | 30 |

| Component * (weight %) | 21 | 22 | 23 | 24 | 25 | 26 | 27 | 28 | 29 | 30 |
|---|---|---|---|---|---|---|---|---|---|---|
| PEBAX™ 7233 | | | | | | | | | | |
| PEBAX™ 6333 | | | | | | | | | | |
| PEBAX™ 5533 | | | | | | | | | | |
| PEBAX™ 4033 | | | | | | | | | | |
| PEBAX™ 3533 | 100 | 98.0 | 97.0 | 79.0 | 78.0 | | | | | |
| PEBAX™ 2533 | | | | | | 100.0 | 98.0 | 97.0 | 79.0 | 78.0 |
| EPOLENE™ C13 | | | | 18.0 | 18.0 | | | | 18.0 | 18.0 |
| LOWINOX™ TBM6 | | 2.0 | 3.0 | 3.0 | 4.0 | | 2.0 | 3.0 | 3.0 | 4.0 |
| MFR (g/10 min): 0 Mrads | 29 | 28 | 30 | 46 | 43 | 51 | 57 | 47 | 68 | 67 |
| MFR: 10 Mrads | 0.4 | 10 | 24 | 26 | 19 | 1 | 20 | 35 | 54 | 39 |
| MFR: 20 Mrads | 0 | 1 | 2 | 16 | 3 | 0 | 2 | 4 | 26 | 29 |

Sixteen compositions were utilized to form tubing layers. Ten compositions were for the outer layer and six compositions were for the inner layer. The outer layer compositions were modified from those of Table 1 by adding 0.5% of antioxidant (Irganox™ 1010, phenolic antioxidant available from Ciba Specialty Chemicals Corporation) and 0.5-3.0% of color concentrates (Wilson™ 50-BU-302 or Wilson™ 50-YE-308, available from PolyOne Corporation). The inner layer compositions were modified from those of Table 2 by adding 0.25% of antioxidant. Two compositions for the outer layer (15 and 16) also contained 5% of wax. These sixteen compositions represented different durometer resins.

All of the compositions listed in Table 3 were converted into dual wall tubing. Each composition was melt blended on a 31.8 mm (1.25 in) Davis Standard extruder and was pelletized. The dual wall tubing was co-extruded using a co-extrusion line to produce tubing having the layers shown in Table 4. In most of the samples both layers of each tubing prototype were made from the same resin. The outer (crosslinked) layers were extruded on a 31.8 mm (1.25 in) Davis Standard extruder at a temperature between 149 °C (300 °F) and 190 °C (374 °F). The inner (uncrosslinked) layers were extruded using a 19.1 mm (0.75 in) C. W. Brabender extruder at a temperature between 163 °C (325 °F) and 204 °C (399 °F). For example, a dual wall tubing (Example 1, Table 4) made from PEBAX™ 5533 (compositions 6 and 8, Table 3) had an extruded outside diameter (OD) of 0.89 mm) (0.035 in) and an extruded inside diameter (ID) of 0.69 mm (0.027 in), and a total average unexpanded wall thickness for the outer layer of 0.051 mm (0.002 in) and a total average unexpanded wall thickness for the inner layer of 0.051 mm (0.002 in). The tubing was irradiated to 25 Mrads using a 0.5 MeV electron beam to an E30 value at 200°C of 0.74 MPa (107 psi) and was expanded in a pressure expander at 177 °C (350°F) to give tubing having an expanded ID of 1.73 mm (0.068 inches) and a recovered ID of 0.686 mm (0.027 inches), an average recovered wall thickness of the outer layer of 0.076 mm (0.003 in), and an average recovered wall thickness of the inner layer of 0.064 mm (0.0025 in).

In similar manner additional dual wall tubing was made from other compositions listed in Table 3. For example, compositions 1 (outer) and 3 (inner) were combined to co-extrude dual wall tubing from PEBAX™ 7233 (Example 2, Table 4). Compositions 4 (outer) and 5 (inner) were co-extruded to a make dual wall tubing from PEBAX™ 6333 (Example 3, Table 4). Compositions 9 (outer) and 10 (inner) were co-extruded to make dual wall tubing from PEBAX™ 4033 (Example 4, Table 4). Compositions 11 (outer) and 12 (inner) were co-extruded to make dual wall tubing from PEBAX™ 3533 (Example 5, Table 4). Compositions 13 (outer) and 14 (inner) were co-extruded to make dual wall tubing from PEBAX™ 2533 (Example 6, Table 4). In addition, one tubing sample was made from two different PEBAX™ resins. The outer layer was made from PEBAX™ 3533 (composition 11) and an inner layer was made from PEBAX™ 4033 (composition 10) (Example 7, Table 4). Finally, in one tubing prototype the layers were reversed. The outer layer (uncrosslinked) was made from PEBAX™ 5333 (composition 8) and the inner layer (crosslinked) was made from PEBAX™ 5333 (composition 7) (Example 8, Table 4). All of these tubing prototypes were irradiated to 25 Mrads using a 0.5 MeV electron beam and were expanded using a pressure expander at 177°C (350°F).

**Table 3**

| Dual Wall Tubing Compositions (% by weight) | | | | | | | | |
|---|---|---|---|---|---|---|---|---|
| | 1 | 2 | 3 | 4 | 5 | 6 | 7 | 8 |
| Layer | Outer | Outer | Inner | Outer | Inner | Outer | Outer | Inner |
| PEBAX™ 7233 | 93.75 | 94.0 | 98.75 | | | | | |
| PEBAX™ 6333 | | | | 92.5 | 98.75 | | | |
| PEBAX™ 5533 | | | | | | 92.5 | 94.0 | 98.75 |
| PEBAX™ 4033 | | | | | | | | |
| PEBAX™ 3533 | | | | | | | | |
| PEBAX™ 2533 | | | | | | | | |
| TAIC | 5.0 | 5.0 | | 5.0 | | 5.0 | 5.0 | |
| EPOLENE™ C13 | | | | | | | | |
| LOWINOX™ TBM6 | | | 1.0 | | 1.0 | | | 1.0 |
| IRGANOX™ 1010 | 0.5 | 0.5 | 0.25 | 0.5 | 0.25 | 0.5 | 0.5 | 0.25 |
| WILSON™ 50-BU-302 | 0.75 | | | 2.0 | | 2.0 | | |
| WILSON™ 50-YE-308 | | 0.5 | | | | | 0.5 | |

| | 9 | 10 | 11 | 12 | 13 | 14 | 15 | 16 |
|---|---|---|---|---|---|---|---|---|
| Layer | Outer | Inner | Outer | Inner | Outer | Inner | Outer | Outer |
| PEBAX™ 7233 | | | | | | | | |
| PEBAX™ 6333 | | | | | | | | |
| PEBAX™ 5533 | | | | | | | | |
| PEBAX™ 4033 | 94.0 | 97.75 | | | | | | |
| PEBAX™ 3533 | | | 91.5 | 78.75 | | | 89.0 | |
| PEBAX™ 2533 | | | | | 94.0 | 78.75 | | 89.0 |
| TAIC | 5.0 | | 5.0 | | 5.0 | | 5.0 | 5.0 |
| EPOLENE™ C13 | | | | 18.0 | | 18.0 | 5.0 | 5.0 |
| LOWINOX™ TBM6 | | 2.0 | | 3.0 | | 3.0 | | |
| IRGANOX™ 1010 | 0.5 | 0.25 | 0.5 | 0.25 | 0.5 | 0.25 | 0.5 | 0.5 |
| WILSON™ 50-BU-302 | | | 3.0 | | | | | |
| WILSON™ 50-YE-308 | 0.5 | | | | 0.5 | | 0.5 | 0.5 |

**Table 4**

| Dual Wall Assemblies | | | | | | | | |
|---|---|---|---|---|---|---|---|---|
| Assembly | 1 | 2 | 3 | 4 | 5 | 6 | 7 | 8 |
| Outer Layer: | | | | | | | | |
| Composition | 6 | 1 | 4 | 9 | 11 | 13 | 11 | 8 |
| Crosslinked | Yes | Yes | Yes | Yes | Yes | Yes | Yes | No |
| Inner Layer | | | | | | | | |
| Composition | 8 | 3 | 5 | 10 | 12 | 14 | 10 | 7 |
| Crosslinked | No | No | No | No | No | No | No | Yes |

The dimensionally recoverable multiple layer systems recited in the examples are suitable for use in the fabrication of reinforced medical devices. The assemblies 100 including the multiple layers are placed adjacent to reinforcing structure 107. The assembly including the reinforcing structure 107 is exposed to heat or is otherwise exposed to conditions to initiate dimensional recovery. As the assembly 100 recovers, the reinforcing structure 107 becomes incorporated into the recovered assembly 300.

## Claims

1. A multi-layered dimensionally heat- recoverable tubing system (100) comprising:
a first crosslinked polymeric layer (103) which is an outer layer of the tubing system;
a second uncrosslinked polymeric layer (105) disposed adjacent to the first layer; and
a reinforcing structure (107) positioned adjacent the second layer;
**characterised in that** the second layer (105) is coextruded with the first layer (103), **in that** the uncrosslinked material of the second polymeric layer has sufficient flowability to allow infiltration by the reinforcing structure and **in that** the first and the second layers are dimensionally heat recoverable and are configured such that the reinforcing structure infiltrates uncrosslinked material of the second layer when the tubing system is dimensionally recovered.

2. The system of claim 1, wherein the first layer comprises a polymer selected from polyolefins, saturated polyesters, polyamides, polyvinyl halides, elastomers, and combinations thereof, preferably wherein the first layer comprises a polymer selected from polyether block amide co-polymers, polyesters, polyurethane elastomers, and combinations thereof.

3. The system of claim 1, wherein the second layer comprises a polymer selected from polyolefins, saturated polyesters, polyamides, polyvinyl halides, elastomers, and combinations thereof, preferably wherein the second layer comprises a polymer selected from polyether block amide co-polymers, polyesters, polyurethane elastomers, and combinations thereof.

4. The system of claim 1, wherein the first layer comprises a crosslinking agent selected from triallyl cyanurate, triallyl isocyanurate, N,N'-m-phenylene-dimaleimide, and combinations thereof.

5. The system of claim 1, wherein the second layer further comprises an antioxidant selected from phenolic antioxidant, thioester, and combinations thereof.

6. The system of claim 1, wherein the second layer comprises an adhesive.

7. A reinforced medical device comprising a multi-layered dimensionally heat-recoverable tubing system according to any preceding claim wherein the a reinforcing structure (107) is incorporated into the second layer.

8. The device of claim 7, comprising a catheter formed from said tubing system.

9. A method for making a reinforced tubular medical device comprising:
providing a crosslinkable tubular first polymeric layer (103);
coextruding an uncrosslinked tubular second polymeric layer (105) adjacent to and within the first layer to form a multiple layer assembly;
exposing the first layer (103) to conditions sufficient to result in crosslinking of the first layer;
expanding the multiple layer assembly to render the multiple layer assembly dimensionally heat recoverable;
positioning a reinforcing structure (107) adjacent to and within the second layer (105); heating the multiple layer assembly to a temperature sufficient to at least partially dimensionally recover the first layer and to incorporate the reinforcing structure into the second layer; and
consolidating the multiple layer assembly to form a reinforced multiple layer device.

10. The method of claim 9, wherein the conditions include irradiation with ionizing radiation, preferably wherein the irradiation includes exposure to a high energy source selected from accelerated electrons, ultraviolet light, X-rays, gamma rays, alpha particles, beta particles, neutrons, and combinations thereof.

11. The method of claim 9, wherein the reinforcing structure is a woven braid.

## Patentansprüche

1. Mehrlagiges, durch Wärme dimensional rückstellbares Schlauchsystem (100), das Folgendes umfasst:
eine erste vernetzte polymere Lage (103), die eine Außenlage des Schlauchsystems ist;
eine zweite unvernetzte polymere Lage (105), die neben der ersten Lage angeordnet ist; und
eine Verstärkungsstruktur (107), die neben der zweiten Lage positioniert ist;
**dadurch gekennzeichnet, dass** die zweite Lage (105) mit der ersten Lage (103) koextrudiert ist, dass das unvernetzte Material der zweiten polymeren Lage ausreichende Fließfähigkeit hat, um eine Infiltration durch die Verstärkungsstruktur zuzulassen, und dadurch, dass die erste und die zweite Lage durch Wärme dimensional rückstellbar und so konfiguriert sind, dass die Verstärkungsstruktur unvernetztes Material der zweiten Lage infiltriert, wenn das Schlauchsystem dimensional zurückgestellt wird.

2. System nach Anspruch 1, wobei die erste Lage ein Polymer umfasst, das aus Polyolefinen, gesättigten Polyestern, Polyamiden, Polyvinylhaliden, Elastomeren und Kombinationen davon ausgewählt ist, wobei die erste Lage vorzugsweise ein Polymer umfasst, das aus Polyether-Blockamid-Copolymeren, Polyestern, Polyurethanelastomeren und Kombinationen davon ausgewählt ist.

3. System nach Anspruch 1, wobei die zweite Lage ein Polymer umfasst, das aus Polyolefinen, gesättigten Polyestern, Polyamiden, Polyvinylhaliden, Elastomeren und Kombinationen davon ausgewählt ist, wobei die zweite Lage vorzugsweise ein Polymer umfasst, das aus Polyether-Blockamid-Copolymeren, Polyestern, Polyurethanelastomeren und Kombinationen davon ausgewählt ist.

4. System nach Anspruch 1, wobei die erste Lage ein Vernetzungsmittel umfasst, das aus Triallylcyanurat, Triallylisocyanurat, N,N'-m-Phenylendimaleimid und Kombinationen davon ausgewählt ist.

5. System nach Anspruch 1, wobei die zweite Lage ferner ein Antioxidationsmittel umfasst, das aus phenolischem Antioxidationsmittel, Thioester und Kombinationen davon ausgewählt ist.

6. System nach Anspruch 1, wobei die zweite Lage einen Klebstoff umfasst.

7. Verstärkte medizinische Vorrichtung, die ein mehrlagiges, durch Wärme dimensional rückstellbares Schlauchsystem nach einem vorherigen Anspruch umfasst, wobei die Verstärkungsstruktur (107) in die zweite Lage integriert ist.

8. Vorrichtung nach Anspruch 7, die einen Katheter umfasst, der aus dem genannten Schlauchsystem gebildet ist.

9. Verfahren zur Herstellung einer verstärkten tubulären medizinischen Vorrichtung, das Folgendes beinhaltet:
Bereitstellen einer vernetzbaren tubulären ersten polymeren Lage (103);
Koextrudieren einer unvernetzten tubulären zweiten polymeren Lage (105) neben und innerhalb der ersten Lage zum Bilden einer mehrlagigen Baugruppe;
Aussetzen der ersten Lage (103) gegenüber Bedingungen, die ausreichen, um zur Vernetzung der ersten Lage zu führen;
Expandieren der mehrlagigen Baugruppe, um die mehrlagige Baugruppe durch Wärme dimensional rückstellbar zu machen;
Positionieren einer Verstärkungsstruktur (107) neben und innerhalb der zweiten Lage (105);
Erhitzen der mehrlagigen Baugruppe auf eine Temperatur, die ausreicht, um die erste Lage wenigstens teilweise dimensional zurückzustellen und die Verstärkungsstruktur in die zweite Lage zu integrieren; und
Konsolidieren der mehrlagigen Baugruppe zum Bilden einer verstärkten mehrlagigen Vorrichtung.

10. Verfahren nach Anspruch 9, wobei die Bedingungen das Bestrahlen mit Ionisierungsstrahlung beinhalten, wobei die Bestrahlung vorzugsweise das Aussetzen gegenüber einer Hochenergiequelle beinhaltet, ausgewählt aus beschleunigten Elektronen, ultraviolettem Licht, Röntgenstrahlen, Gammastrahlen, Alphapartikeln, Betapartikeln, Neutronen und Kombinationen davon.

11. Verfahren nach Anspruch 9, wobei die Verstärkungsstruktur ein Geflecht ist.

## Revendications

1. Système de tubage multicouches pouvant reprendre ses dimensions à chaud (100) comprenant :
une première couche polymérique réticulée (103) qui est une couche externe du système de tubage ;
une seconde couche polymérique non réticulée (105) disposée adjacente à la première couche ; et
une structure de renforcement (107) positionnée adjacente à la seconde couche ;
**caractérisé en ce que** la seconde couche (105) est coextrudée avec la première couche (103), **en ce que** la matière non réticulée de la seconde couche polymérique a une fluidité suffisante pour permettre l'infiltration par la structure de renforcement et **en ce que** les première et seconde couches peuvent reprendre leurs dimensions à chaud et sont configurées de telle sorte que la structure de renforcement infiltre la matière non réticulée de la seconde couche quand le système de tubage a repris ses dimensions.

2. Système selon la revendication 1, dans lequel la première couche comprend un polymère sélectionné parmi les polyoléfines, polyesters saturés, polyamides, halogénures de polyvinyle, élastomères et des combinaisons de ceux-ci, de préférence dans lequel la première couche comprend un polymère sélectionné parmi les copolymères de polyester bloc amide, polyesters, élastomères de polyuréthane et des combinaisons de ceux-ci.

3. Système selon la revendication 1, dans lequel la seconde couche comprend un polymère sélectionné parmi les polyoléfines, polyesters saturés, polyamides, halogénures de polyvinyle, élastomères et des combinaisons de ceux-ci, de préférence dans lequel la seconde couche comprend un polymère sélectionné parmi les copolymères de polyester bloc amide, polyesters, élastomères de polyuréthane et des combinaisons de ceux-ci.

4. Système selon la revendication 1, dans lequel la première couche comprend un agent de réticulation sélectionné parmi le cyanurate de triallyle, l'isocyanurate de triallyle, le N,N'-m-phénylène-dimaléimide, et des combinaisons de ceux-ci.

5. Système selon la revendication 1, dans lequel la seconde couche comprend en outre un antioxydant sélectionné parmi un antioxydant phénolique, un thioester, et des combinaisons de ceux-ci.

6. Système selon la revendication 1, dans lequel la seconde couche comprend un adhésif.

7. Dispositif médical renforcé comprenant un système de tubage multicouches pouvant reprendre ses dimensions à chaud selon l'une quelconque des revendications précédentes dans lequel une structure de renforcement (107) est incorporée dans la seconde couche.

8. Dispositif selon la revendication 7, comprenant un cathéter formé à partir dudit système de tubage.

9. Procédé de fabrication d'un dispositif médical tubulaire renforcé comprenant :
la fourniture d'une première couche polymérique tubulaire réticulable (103) ;
la coextrusion d'une seconde couche polymérique tubulaire non réticulée (105) adjacente à la première couche et à l'intérieur de celle-ci pour former un ensemble multicouches ;
l'exposition de la première couche (103) à des conditions suffisantes pour entraîner la réticulation de la première couche ;
l'expansion de l'ensemble multicouches pour faire de l'ensemble multicouches un ensemble multicouches pouvant reprendre ses dimensions à chaud ;
le positionnement d'une structure de renforcement (107) adjacente à la seconde couche (105) et à l'intérieur de celle-ci ;
le chauffage de l'ensemble multicouches à une température suffisante pour que la première couche reprenne au moins partiellement ses dimensions et pour incorporer la structure de renforcement dans la seconde couche ; et
la consolidation de l'ensemble multicouches pour former un dispositif multicouches renforcé.

10. Procédé selon la revendication 9, dans lequel les conditions comportent une irradiation avec un rayonnement ionisant, de préférence dans lequel l'irradiation comporte l'exposition à une source de haute énergie sélectionnée parmi les électrons accélérés, la lumière ultraviolette, les rayons X, les rayons gamma, les particules alpha, les particules bêta, les neutrons, et des combinaisons de ceux-ci.

11. Procédé selon la revendication 9, dans lequel la structure de renforcement est une tresse tissée.
